# EUROPEAN PATENT SPECIFICATION

(11) **EP 1 214 061 B1**
(45) Date of publication and mention of the grant of the patent: **23.06.2004**
(21) Application number: 00964144.0
(22) Date of filing: 29.08.2000
(51) Int. Cl.: A61K 31/337, A61P 35/00

(54) **USE OF DOCETAXEL FOR TREATING HEPATOCELLULAR CARCINOMA**
VERWENDUNG VON DOCETAXEL FÜR DIE BEHANDLUNG VON HEPATOZELLULÄRE KREBS
UTILISATION DE DOCETAXEL POUR LE TRAITEMENT DU CARCINOME HEPATOCELLULAIRE

(30) Priority: 31.08.1999 GB 9920548
(43) Date of publication of application: 19.06.2002
(73) Proprietor: Aventis Pharma S.A., 92160 Antony (FR)
(72) Inventor: CHI, Chin-Wen, Shih-Pai, Taipei 112 (TW); LIN, Heng-Liang, Taipei 111 (TW); LIU, Tsung-Yun, Taipei 106 (TW); LUI, Wing-Yiu, Taipei 112 (TW); CHAU, Gar-Yang, Taipei 112 (TW)
(74) Representative: Le Pennec, Magali
(86) International application number: PCT/EP2000/008782
(87) International publication number: WO 2001/015675

(56) References cited:
- WO-A-00/38786
- US-A- 5 436 243
- US-A- 5 670 536
- CLARK JEFFREY ET AL: "A Phase I Trial of Gemcitabine (G) Given Concurrently with Docetaxel (D) in Patients with Metastatic Solid Tumors. (Meeting abstract)." PROC ANNU MEET AM SOC CLIN ONCOL, (1999). VOL. 18, PP. A792., XP000986303 Dana-Farber/Partners CancerCare, Boston, MA.
- RYAN DAVID P ET AL: "A phase I study of gemcitabine and docetaxel in patients with metastatic solid tumors." CANCER, vol. 88, no. 1, 1 January 2000 (2000-01-01), pages 180-185, XP000983980 ISSN: 0008-543X

## Description

This invention relates to treatment of hepatocellular carcinoma.

Hepatocellular carcinoma (HCC) is one of the most common cancers in Southeast Asia and African countries. In Taiwan, HCC is the leading cause of death in male cancer patients. The survival rate of HCC patients is very low. This is mainly due to lack of effective treatments. Irradiation and chemotherapies have not so far proved to be satisfactory; surgery is the most effective treatment for HCC. However, surgery is only appropriate for patients with small resectable tumours.

Recently, antimitotic drugs such as paclitaxel have received renewed interest. Paclitaxel was originally isolated from the bark of the Yew tree. The antitumour effect of paclitaxel has been known since 1971. Paclitaxel inhibits tumour cell division by its action on microtubule assembly. *In vitro* analyses using tumour cells have revealed that paclitaxel arrests cells mainly in the G2/M phase of the cell cycle (Schiff PB and Horwitz SB, Proc. Natl. Acad. Sci **77**, 1561 - 1565, 1980). Recent studies have shown that paclitaxel is effective against various malignant tumour cells such as brain tumour, gastric and prostate cancer, breast cancer, melanoma and ovarian cancer.

However, paclitaxel is not effective against hepatocellular carcinoma. A phase II clinical trial of paclitaxel for HCC patients is reported in British Journal of Cancer, **78** (1), 34-39, 1998. That article concludes that paclitaxel had no significant anti-cancer effect in HCC patients.

As explained above, the cytotoxic effect of paclitaxel has been found to be cell cycle dependent, with cell cycle arrest occurring mainly at the G2/M phase. However, it has now been found that docetaxel can achieve non cell cycle dependent cytotoxicity in HCC cells. This indicates that the cytotoxic effect of docetaxel on HCC cells is achieved by a different mechanism from that of paclitaxel. Further, the *in vitro* activity of docetaxel against HCC cells is significantly higher than that of paclitaxel at concentrations of up to 1µM. Given the highly cytotoxic nature of the taxoids, an increased activity at low concentration suggests that docetaxel, unlike paclitaxel, will be of practical use in the clinical treatment of hepatocellular carcinoma.

Accordingly, the present invention provides the use of docetaxel or a hydrate thereof in the manufacture of a medicament for use in the treatment of hepatocellular carcinoma.

Docetaxel is a known compound. It has the formula 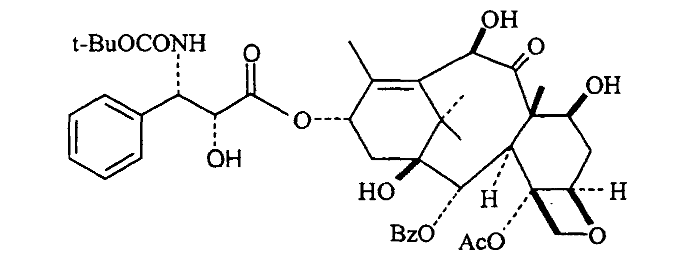 Processes for the preparation of docetaxel are described in EP-A-253738 and EP-A-336841.

Docetaxel may be used, for example, in anhydrous form or as a hydrate. As used herein, references to docetaxel include references to hydrates thereof.

Docetaxel hydrates can be prepared by dissolving anhydrous docetaxel in an organic solvent such as acetone, ethanol, acetonitrile or N,N-dimethylformamide, and by recrystallising docetaxel hydrate by adding the thus obtained solution to water. A docetaxel hydrate is typically a dihydrate, a trihydrate or a tetrahydrate. In particular, the trihydrate has been found to be particularly stable, and docetaxel trihydrate is accordingly preferred. Docetaxel trihydrate may be prepared by the processes set out in EP-A-770070.

Docetaxel is unexpectedly active against hepatocellular carcinomas. In particular, it can be used to treat liver cell carcinomas, fibrolamellar variants and mixed hepatocellular cholangiocarcinomas.

In the present invention, docetaxel may be administered by any conventional route known for the administration of docetaxel. Thus; it may, for example, be administered parenterally. Typically, it is administered intravenously, preferably by intravenous infusion.

In the present invention, docetaxel is typically formulated for administration as a pharmaceutically acceptable composition containing docetaxel and a pharmaceutically acceptable carrier or diluent. Suitable carriers and diluents include non-toxic solvents and suspension media, for example sterile aqueous media. Preferably, the compositions take the form of aqueous solutions or suspensions, for example, solutions suitable for injection or infusion, which can contain emulsifying agents, colourings, preservatives or stabilizers.

Pharmaceutical compositions suitable for parenteral administration include sterile aqueous or non-aqueous solutions or suspensions. Suitable sterile non-aqueous solutions and suspensions include solutions and suspensions in natural vegetable oils such as olive oil, sesame oil or liquid petroleum or in injectable organic esters such as ethyl oleate. Suitable sterile aqueous solutions include solutions of docetaxel in water. Typically, the pH of sterile aqueous solutions suitable for parenteral administration is appropriately adjusted. Further, such sterile aqueous solutions are generally made isotonic, for example with a sufficient amount of sodium chloride or glucose. It is particularly preferred that solutions suitable for administration by infusion have a pH similar to that of the blood and are made isotonic.

Sterilization may be carried out by heating or by any other means which does not adversely affect the composition.

Pharmaceutical compositions containing docetaxel suitable for use in the present invention may further comprise a surfactant. Preferred surfactants are polysorbates, polyoxyethylene glycol esters and ester-ethers of polyethylene glycol and castor oils. Examples of suitable surfactants, and of pharmaceutical compositions containing the surfactants, can be found in AU-A-666859.

Docetaxel may also be formulated for use in the present invention as a lyophilized composition. Such lyophilized compositions have good physical and chemical stability and can therefore be stored for long periods. Lyophilised compositions containing docetaxel may be prepared by lyophilising an aqueous solution of docetaxel by standard techniques. They may further comprise bulking agents such as lactose. They may also comprise tonicity adjustment agents such as sugars and polymers. Examples of suitable tonicity adjustment agents include glucose, dextrose and mannitol and polymers, for example polyvinylpyrrolidone.

A lyophilized composition may be redissolved at the time of use in any compatible and pharmaceutically acceptable injectable medium. The lyophilizate may be advantageously taken up with injection grade double-distilled water, in a volume equivalent to the initial volume of the solution to be lyophilized.

A pharmaceutical composition containing docetaxel suitable for use in the present invention typically contains at least 0.01% by weight of therapeutically active product. Generally, a pharmaceutical composition contains from 0.01 to 1000 mg, preferably from 0.1 to 500 mg, of therapeutically active product.

Preferably, a solution suitable for intravenous injection contains from 38 to 42, more preferably around 40 mg/ml of active product. Typically, such solutions are provided in vials containing 20 mg or 80 mg of active product.

Preferably, a solution suitable for infusion contains from 0.1 to 11, preferably from 0.1 to 10, more preferably from 0.3 to 0.9 mg/ml of active product.

Therapeutic treatment with docetaxel according to the present invention may be performed concurrently with other therapeutic treatments including treatment with other antineoplastic drugs, monoclonal antibodies, immunotherapy or radiotherapy or biological response modifiers. Suitable biological response modifiers include lymphokines and cytokines such as interleukins, interferons (α, β or δ) and TNF. Other chemotherapeutic agents which are useful in the treatment of disorders due to abnormal cell proliferation include alkylating agents, for instance nitrogen mustards such as mechlorethamine, cyclophosphamide, melphalan and chlorambucil, alkyl sulphonates such as busulfan, nitrosoureas such as carmustine, lomustine, semustine and streptozocin, triazenes such as dacarbazine, antimetabolites such as folic acid analogues, for instance methotrexate, pyrimidine analogues such as fluorouracil and cytarabine, purine analogues such as mercaptopurine and thioguanine, natural products, for instance vinca alkaloids such as vinblastine, vincristine and vindesine, epipodophyllotoxins such as etoposide and teniposide, antibiotics such as dactinomycin, daunorubicin, doxorubicin, bleomycin, plicamycin and mitomycin, enzymes such as L-asparaginase, various agents such as coordination complexes of platinum, for instance cisplatin, substituted ureas such as hydroxyurea, methylhydrazine derivatives such as procarbazine, adrenocortical suppressants such as mitotane and aminoglutethimide, hormones and antagonists such as adrenocorticosteroids such as prednisone, progestins such as hydroxyprogesterone caproate, methoxyprogesterone acetate and megestrol acetate, oestrogens such as diethylstilboestrol and ethynyloestradiol, antioestrogens such as tamoxifen, and androgens such as testosterone propionate and fluoxymesterone.

Concurrent treatment with cyclophosphamide, 5-fluorouracil, etoposide, vinorelbine or methotrexate is preferred, as synegism between these compounds and docetaxel may be achieved. Further, 2-methoxyestradiol is active against hepatocellular carcinomas and has been found to be well tolerated after 1 month of daily treatment in mice (Klauber *et al,* Cancer Research, **57**, 81-86, 1997). Concurrent treatment with 2-methoxyestradiol is therefore also preferred, particularly when chronic treatment is required.

In the present invention, docetaxel is administered at a dosage which permits the treatment of hepatocellular carcinoma. The dosage varies according to the route of administration and the physical characteristics of the patient. Suitable dosages include those which are therapeutically effective for the treatment of disorders due to abnormal cell proliferation. Docetaxel may be administered as often as necessary to obtain the desired therapeutic effect.

A typical dose of docetaxel for the treatment of a human is from 50 to 150, preferably 60 to 100, more preferably around 100 mg docetaxel/m² of surface area of the patient's skin. When docetaxel is administered by infusion, the rate of infusion is typically from 1 to 200, preferably around 100 mg/m² docetaxel per hour.

The above dose may be repeated as required. Typically, it is repeated daily or weekly. Preferably, it is repeated every 3 weeks. For example, docetaxel may be administered at a dose of around 100 mg/m² as an intravenous infusion over 1 hour every 3 weeks.

The following Example illustrates the invention.

### EXAMPLE

### Materials and Methods

Unless otherwise indicated, the methods used are standard biochemical techniques. The cell lines used are all commercially available.

### Cell Culture

The experiments detailed below involve human hepatoma cell lines Hep3B (ATCC designation HB 8064), HepG2 (ATCC designation HB 8065) and HA22T/VGH, and murine hepatoma cell line Hepa 1-6. These cells were cultured in DMEM (GIBCO, BRL) containing 10% fetal bovine serum (Hyclone), 0.01 mg/ml gentamycin and 0.1 mM non-essential amino acid. Cells were grown in a CO₂ incubator at 37°C, with 5% CO₂ and 95% filtered air.

### Drug Treatment

In the experiments detailed below, the above hepatoma cells were treated with different concentrations of paclitaxel (0.001-10 µM) and docetaxel (0.001-10µM) for 24 hours and 72 hours. Paclitaxel was dissolved in dimethylsulfoxide (DMSO) and docetaxel was dissolved in ethanol as stock solutions. The final concentration of vehicle was less than 0.1%.

### Cell Viability Study: MTT assay

Cells were cultured in a 96 well cell culture cluster (COSTAR) at a density of 4x10⁴ cells/ml. After drug treatment for 24 hours or 72 hours, medium was discarded and replaced with an equal volume (100µl) of fresh medium containing MTT (0.456 mg/ml; 3-[4,5-Dimethylthiazol-2-yl]-2,5-diphenyl-tetrazolium bromide) and incubated for 1.5 hours at 37°C. The fresh medium was then discarded, and 100 µl DMSO was then added. Cell viability was determined by colorimetric comparison by reading OD values from a microplate reader (SPECTRA MAX 250) at an absorption wavelength of 570 nm.

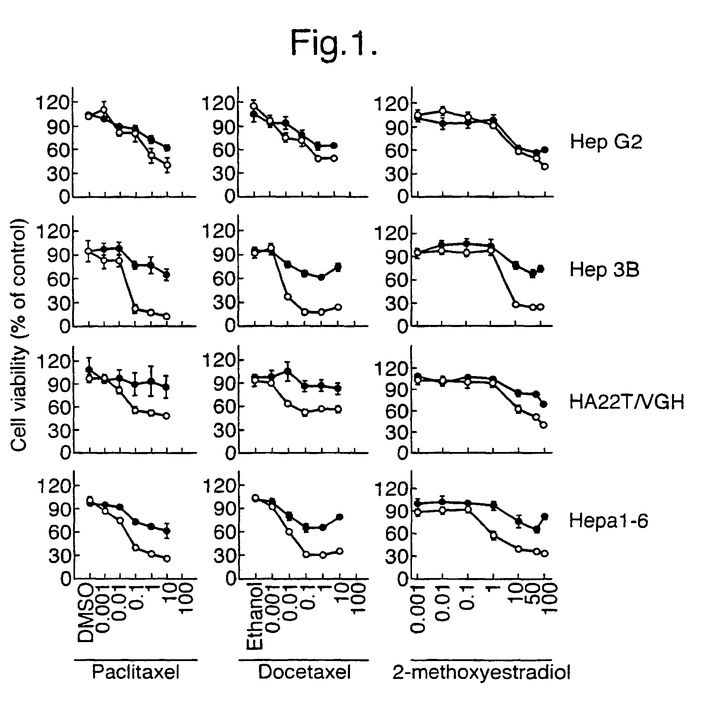

The results are shown in Figure 1, in which filled circles represent data following treatment for 24 hours and open circles represent data following treatment for 72 hours. Data are the mean ± standard error of mean from duplicate samples of three independent experiments.

### Propidium Iodide (PI) Exclusion Assay

Cells were grown on 5-cm² flasks (CORNING) and treated with paclitaxel and docetaxel as set out above. Propidium iodide (10 µg/ml) was then added for 15 minutes incubation at 37°C. Then, medium was collected prior to harvest of the adherent cells. Both suspended and attached cells were collected and resuspended with 500 µl PBS for flow cytometry analysis as set out below. Debris signals were removed by FSC-SSC gating.

### Flow Cytometric Analysis of DNA Content

Lysing buffer (0.5% Triton X-100, 0.2 µg/ml Na₂EDTA.2H₂O, and 1% bovine serum albumin in PBS) was added to the cell pellets which were then left on ice for 15 minutes. 100% methanol pre-cooled to -20°C was then added to the mixture, which was then centrifuged at 300 xg for 5 minutes. The supernatant was discarded and the cell pellet was washed with PBS. The washed pellet was stained with a DNA staining solution (50 µg/ml propidium iodide, and 5 kunitz/ml of RNase A) for 30 minutes at 4°C in the dark. The DNA content of each cell was measured using a Becton Dickinson FACS*Calibur* flow cytometer as set out below.

### Flow Cytometry

Cells (10000) were analyzed on a Becton Dickinson FACS*Calibur* flow cytometer using an argon-ion laser (15 mWatt) with incident beam at 488 nm. For PI exclusion assay, red fluorescence was collected through a 585 nm filter and the cell debris signals were removed by FSC-SSC gating. Data were acquired and analyzed using FACS/CELLQuest software on a Power Macintosh 7600/120 computer. Apoptotic cells and cells at specific cycle phases were determined by ModFit LT software.

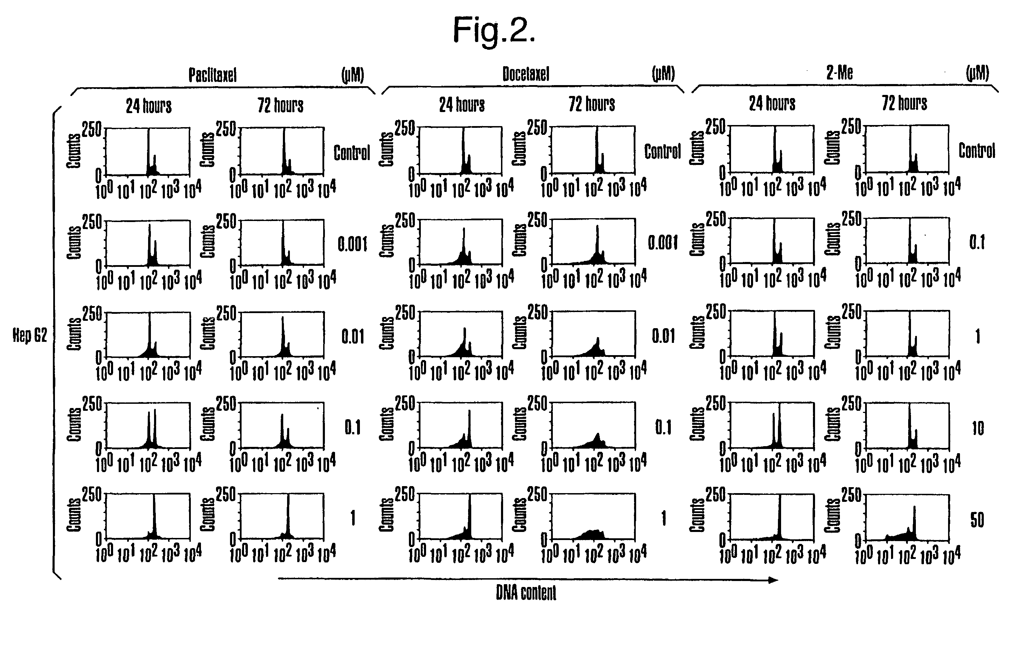

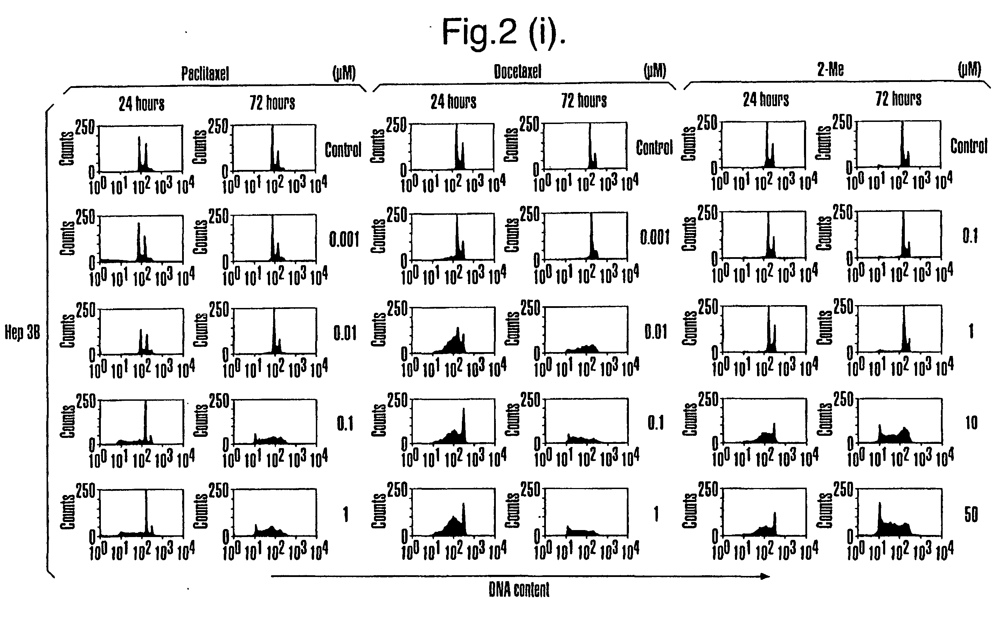

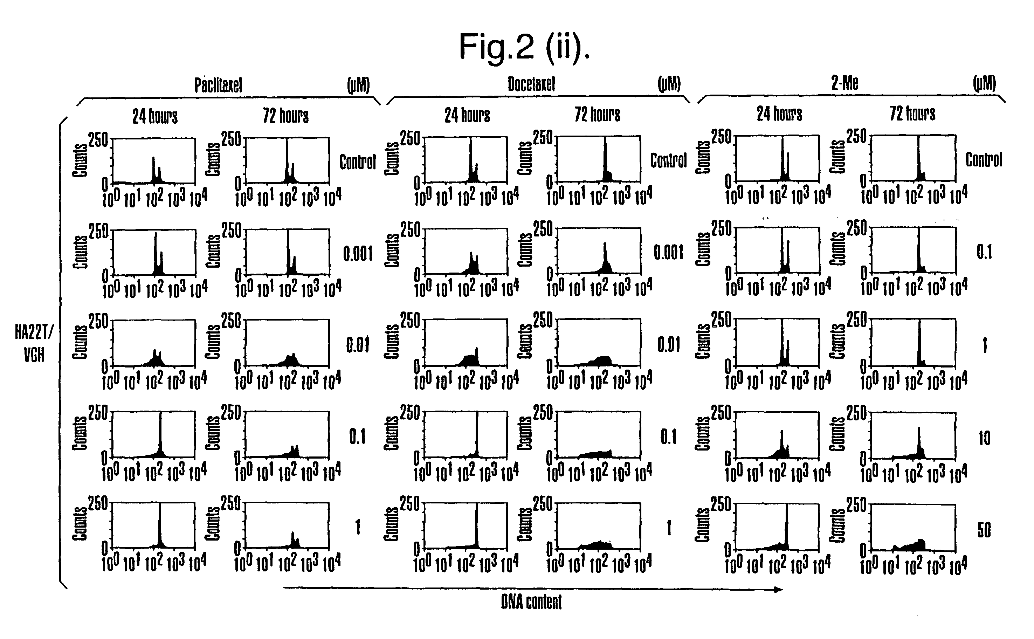

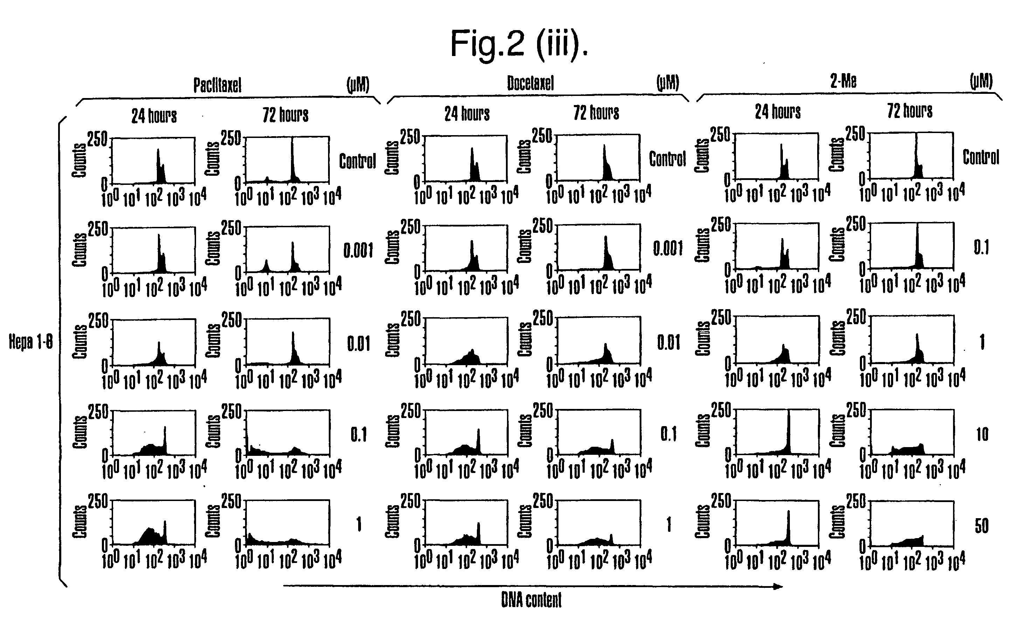

The results of the flow cytometry are shown in Tables 1 and 2 and in Figure 2. Table 1 gives figures for cell membrane permeability of the hepatoma cells, following treatment with paclitaxel and docetaxel. Table 2 details the percentage of apoptotic (sub-G0/G1) cells found after paclitaxel and docetaxel treatment. Figure 2 shows a DNA histogram analysis detailing the effect of paclitaxel and docetaxel on cell cycle progression.

**TABLE 1**

| *Cell membrane permeability of hepatoma cells after treatment with paclitaxel and docetaxel.* | | | | | | |
|---|---|---|---|---|---|---|
| | Paclitaxel (µM) | | | Docetaxel (µM) | | |
| | 0.01 | 0.1 | 1 | 0.01 | 0.1 | 1 |
| | | | | | | |
| **Hep G2** | | | | | | |
| 24 hrs | 93.63±1.1 | 85.71±6.8 | 66.71±7.2 | 94.86±1.3 | 85.49±1.2 | 81.24±3.2 |
| 72 hrs | 56.58±28.7 | 43.79±11.7 | 13.27±4.3 | 61.06±9.6 | 40.03±9.0 | 27.42±8.8 |

| **Hep 3B** | | | | | | |
|---|---|---|---|---|---|---|
| 24 hrs | 77.35±11.7 | 63.50±4.0 | 52.28±4.1 | 93.80±10.7 | 57.41±6.8 | 57.39±4.3 |
| 72 hrs | 57.00±7.9 | 8.09±2.3 | 1.90±0.3 | 36.81±14.7 | 36.25±13.5 | 20.25±14.4 |

| **HA22T/VGH** | | | | | | |
|---|---|---|---|---|---|---|
| 24 hrs | 94.08±18.6 | 40.03±7.8 | 34.24±8.3 | 98.66±9.0 | 38.71±11.2 | 40.79±5.0 |
| 72 hrs | 92.58±21.3 | 93.38±32.5 | 49.32±8.3 | 55.44±5.6 | 21.24±0.4 | 22.03±3.1 |

| **Hepa 1-6** | | | | | | |
|---|---|---|---|---|---|---|
| 24 hrs | 93.17±3.8 | 67.20±4.4 | 62.65±7.6 | 94.45±1.9 | 83.35±7.2 | 81.88±8.7 |
| 72 hrs | 62.95±5.6 | 27.79±1.3 | 15.51±1.0 | 77.18±1.4 | 43.94±3.4 | 38.90±4.2 |

*Data are mean ± standard error of mean from duplicated samples of at least three independent experiments. Cells were treated with drugs for 24-72 hours, and membrane permeability was measured by flowcytometric analysis of propidium exclusion in viable hepatoma cells. Data are the percentage of cells with intact cell membrane as compared to control.*

**TABLE 2**

| *Paclitaxel and Docetaxel induced apoptosis* | | | | | | | |
|---|---|---|---|---|---|---|---|
| | | Paclitaxel (mM) | | | Docetaxel (mM) | | |
| | | 0.01 | 0.1 | 1 | 0.01 | 0.1 | 1 |
| **Hep G2** | 24 hrs | | | | 45.24 | 38.77 | 28.33 |
| | 72 hrs | | | | 42.45 | 42.44 | 56.66 |
| | | | | | | | |
| **Hep 3B** | 24 hrs | | | | 59.14 | 58.67 | 65.74 |
| | 72 hrs | | 38.37 | 47.01 | 64.12 | 81.66 | 79.33 |
| | | | | | | | |
| **HA22T/** | 24 hrs | | | | 41.75 | 18.61 | 22.94 |
| **VGH** | 72 hrs | | 0 | 0 | 56.64 | 58.61 | 60.98 |
| | | | | | | | |
| **Hepa 1-6** | 24 hrs | 24.02 | 55.64 | 64.38 | 52.81 | 50.76 | 53.80 |
| | 72 hrs | | N/A | N/A | 31.25 | 53.95 | 62.49 |

*Figures are % of apoptotic (sub-G0*/*G1) cells as determined by flow cytometry. DNA Fragmentation Electrophoresis Analysis*

DNA fragmentation assessment was according to the method of Herrmann *et al*, Nucleic Acids Res., **22**, 5506-5507, 1994.

Briefly, HEP G2 cells (2x10⁷) were treated for 72 hours with paclitaxel and docetaxel as set out above and centrifuged. The thus obtained cell pellets were resuspended with NP-40 lysis buffer (1% NP-40 in 20 mM EDTA, 50 mM Tris-HCl, pH 7.5). After lysis of cells for a few seconds, the supernatants were collected (5 minutes at 1600 x g). The extraction was repeated with the same lysis buffer. SDS (final concentration 1%) and RNase were added (final concentration 2.5 µg/µl) to supernatants and incubated for 2 hours at 56°C followed by digestion with proteinase K (2.5 µg/µl) for 2 hours at 37°C. Then, the mixtures were added to 10 M ammonium acetate prior to 100% ethanol precipitation for 30 minutes at -20°C. The DNA was collected by centrifiguration (10 min at 12000 x g) followed by electrophoresis on 1.5% agarose gel.

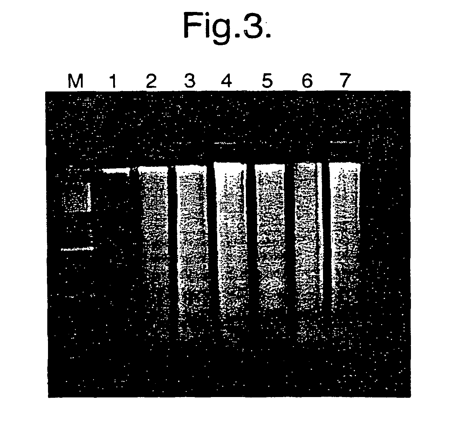

The results are shown in Figure 3. In Figure 3, M is a 100 base pair marker. Lane 1 shows medium control. Lanes 2 and 3 show mean paclitaxel (0.1 and 1 µM) treatment groups. Lanes 4 and 5 show mean docetaxel (0.1 and 1 µM) treatment groups.

### Results

### Cell Viability Studies

Figure I shows the dose-dependent effect of paclitaxel and docetaxel on cell viability in hepatoma cell lines (Hep G2, Hep 3B, HA22T/VGH and Hepa 1-6). As is evident from Figure 1, docetaxel achieved a decreased viability at 0.01 and 0.1µM in nearly every case.

In Hep G2 cells, cell viability showed a decreasing trend after treatment with paclitaxel or docetaxel. The viability of Hep G2 cells was 61.81% and 39.45% of control for paclitaxel (10 µM) groups at 24 and 72 hours, respectively. For docetaxel treated Hep G2 cells, maximal reduction of viability was observed at 1µM docetaxel, no further decrease in viability was found at 10µM docetaxel. The viability was 65.03% and 48.99% for 1 µM docetaxel treated cells at 24 and 72 hours, respectively.

In Hep 3B cells it is noteworthy that significant reduction of viability (37.06%) was observed after 0.01 µM docetaxel treatment for 72 hours.

In docetaxel treated Hepa 1-6 cells, maximum cytotoxicity (65.34% and 30.71%) was found at 1 µM docetaxel treatment groups at 24 and 72 hours, respectively.

### Propidium Iodide (PI) Exclusion Assay

Table 1 shows that membrane permeability of Hep G2 cells and Hep 3B cells following treatment with paclitaxel and docetaxel was dose and time dependent.

For HA22T/VGH cells, less increase in membrane permeability was observed after paclitaxel (0.01-1 µM) treatment for 72 hours as compared to that of docetaxel groups. It is noteworthy that only 55.44% of cells had intact membranes after 0.01 µM docetaxel treatment for 72 hours, whereas, following the same dose of paclitaxel, 92.58% of treated cells had intact membranes.

### Cell Cycle Analysis

Figure 2 shows that 1 µM paclitaxel-treated Hep G2 cells for 24 hours resulted in an obvious G2/M phase arrest. Similar DNA histograms were observed at 72 hours after exposure.

As shown in Table 2, apoptotic cells (sub-G0/G1) were found after treatment with 0.001 µM, 0.01 µM, 0.1 µM and 1 µM docetaxel for 24 hours with apoptotic percentages of 31.02%, 45.24%, 38.77% and 28.33%, respectively. At 72 hours after docetaxel treatment (0.001-1 µM), the apoptotic percentages were 21.92%, 42.45%, 42.44% and 56.66%, respectively.

In Hep 3B cells, 0.1 µM or 1 µM paclitaxel treatment for 24 hours resulted in G2/M arrest and incubation with 0.1 µM or 1 µM paclitaxel for 72 hours resulted in increased sub-G0/G1 percentages to 38.37% or 47.01%, respectively. In contrast, 0.01 µM, 0.1 µM or 1 µM docetaxel treated Hep 3B cells for 24 hours or 72 hours gave rise to high levels of sub-G0/G1 populations of 59.14%, 58.69% and 65.74% at 24 hours and 64.12%, 81.66% and 79.33% at 72 hours.

In HA22T/VGH cells, the increasing concentrations of paclitaxel (0.001 µM to 1 µM) correlated with the elevated percentage of G2/M cells at 24 hours. No significant sub-G0/G1 population was observed in 0.1 µM or 1 µM paclitaxel treatment groups at 72 hours. In contrast, it is significant that 0.01 µM docetaxel-treated HA22T/VGH cells at 24 hours had a higher sub G0/G1 percentage (41.75 %) than 0.1 µM (18.61 %) or 1 µM (22.94%) docetaxel groups. When cells were treated with docetaxel for 72 hours, significant sub G0/G1 percentages were found in 0.01 µM (56.64 %), 0.1 µM (58.61 %) and 1 µM (60.98 %) docetaxel-treated HA22T/VGH cells.

For Hepa 1-6 cells, paclitaxel treatment (0.01, 0.1 or 1µM) for 24 hours resulted in increased formation of sub-G0/G1 populations (24.02 %, 55.64 % or 64.38 %, respectively), and G2/M phase arrest was observed in 0.1 µM and 1 µM paclitaxel treatment groups. When Hepa 1-6 cells were treated with 0.1 µM and 1 µM paclitaxel for 72 hours, most of the cells were dead and there was no obvious cell cycle profile. Docetaxel treatment (0.01 µM, 0.1 µM and 1 µM) of Hepa 1-6 cells resulted in formation of sub-G0/G1 cells (52.81 %, 50.76 % and 53.8 % at 24 hours and 31.25 %, 53.95 % and 62.49 % at 72 hours, respectively).

### DNA Fragmentation Analysis

Figure 3 shows that paclitaxel ( 0.1 and 1 µM) and docetaxel (0.1 and 1 µM) treatment induced DNA fragmentation in Hep G2 cells.

## Claims

1. Use of a composition for the preparation of a drug for the treatment of hepatocellular carcinoma said composition comprising, as active ingredient, docetaxel or a hydrate thereof.

2. Use of a composition according to claim 1, wherein the hydrate is the trihydrate.

3. Use of a composition according to claim 1 or 2, for the preparation of a drug for treating hepartocarcinoma where the drug is to be used by parenteral administration.

4. Use of a composition according to claim 3, which is suitable for administration by infusion and contains from 0.1 to 11 mg/ml of active compound.

## Patentansprüche

1. Verwendung einer Zusammensetzung zur Herstellung eines Arzneimittels zur Behandlung von hepatozellulärem Krebs, wobei die besagte Zusammensetzung als Wirkstoff Docetaxel oder ein Docetaxel-Hydrat enthält.

2. Verwendung einer Zusammensetzung nach Anspruch 1, bei der das Hydrat das Trihydrat ist.

3. Verwendung einer Zusammensetzung nach Anspruch 1 oder 2 zur Herstellung eines Arzneimittels zur Behandlung von hepatozellulärem Krebs, wobei dieses Arzneimittel durch parenterale Verabreichung anzuwenden ist.

4. Verwendung einer Zusammensetzung nach Anspruch 3, die zur Verabreichung per Infusion geeignet ist und deren Wirkstoffgehalt 0,1 bis 11 mg/ml beträgt.

## Revendications

1. Utilisation d'une composition pour la préparation d'un médicament destiné au traitement du carcinome hépatocellulaire, ladite composition comprenant, en tant qu'ingrédient actif, du docétaxel ou un hydrate de celui-ci.

2. Utilisation d'une composition selon la revendication 1, dans laquelle l'hydrate est le trihydrate.

3. Utilisation d'une composition selon la revendication 1 ou 2, pour la préparation d'un médicament destiné au traitement de l'hépatocarcinome, où le médicament est destiné à être utilisé par administration par voie parentérale.

4. Utilisation d'une composition selon la revendication 3, qui est appropriée pour l'administration par perfusion et contient de 0,1 à 11 mg/ml de composé actif.
